(19) Europäisches Patentamt
European Patent Office
Office européen des brevets

(11) Numéro de publication : **0 049 658**
**B1**

(12) **FASCICULE DE BREVET EUROPÉEN**

(45) Date de publication du fascicule du brevet : **13.06.84**

(51) Int. Cl.³ : **C 07 D217/26**, C 07 D209/42, A 61 K 31/40, A 61 K 31/47

(21) Numéro de dépôt : **81401501.2**

(22) Date de dépôt : **29.09.81**

(54) **Iminodiacides substitués, leur préparation et compositions pharmaceutiques les contenant.**

(30) Priorité : **02.10.80 FR 8021095**
**07.04.81 FR 8106916**

(43) Date de publication de la demande : **14.04.82 Bulletin 82/15**

(45) Mention de la délivrance du brevet : **13.06.84 Bulletin 84/24**

(84) Etats contractants désignés : **AT BE CH DE FR GB IT LI LU NL SE**

(56) Documents cités :
**EP-A- 0 012 401**
**EP-A- 0 012 845**
**EP-A- 0 018 104**
**EP-A- 0 018 549**
**EP-A- 0 024 852**
**EP-A- 0 031 741**
**EP-A- 0 046 953**
**FR-A- 2 448 533**
**FR-A- 2 456 733**
**US-A- 4 251 444**
**Le dossier contient des informations techniques présentées postérieurement au dépôt de la demande et ne figurant pas dans le présent fascicule.**

(73) Titulaire : **ADIR**
**22, rue Garnier**
**F-92200 Neuilly S/Seine (FR)**

(72) Inventeur : **Remond, Georges**
**9, av. des Etats-Unis**
**F-78000 Versailles (FR)**
Inventeur : **Laubie, Michel**
**35, av. Foch**
**F-92420 Vaucresson (FR)**
Inventeur : **Vincent, Michel**
**8, Allée du Prunier Hardy**
**F-92220 Bagneux (FR)**

**EP 0 049 658 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux iminodiacides substitués, plus précisément des acides perhydroindoledicarboxyliques substitués, leur préparation et les compositions pharmaceutiques les contenant.

Spécifiquement, l'invention concerne les composés répondant à la formule générale :

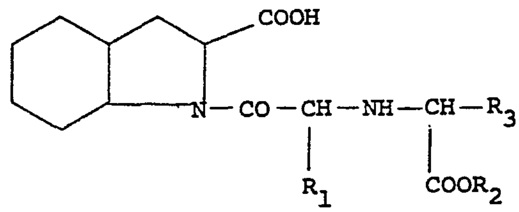

dans laquelle

$R_1$ représente un groupe alkyle inférieur de 1 à 4 atomes de carbone,

$R_2$ représente un atome d'hydrogène ou un groupe alkyle de 1 à 4 atomes de carbone,

$R_3$ représente un groupe alkyle linéaire ou ramifié, ou mono- ou di-cycloalkyl-alkyle, chacun ayant au plus au total 9 atomes de carbone, ou bien un groupe alkyle substitué de formule :

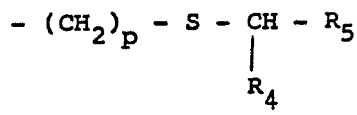

avec

$R_4$ = H, alkyle inférieur ($C_1$ à $C_4$) ou cycloalkyle (de $C_3$ à $C_6$)

$R_5$ = H, alkyle inférieur ($C_1$ à $C_4$), cycloalkyle ($C_3$ à $C_6$) ou alcoxycarbonyle, et

p = 1 ou 2.

Les composés de l'invention comportent au moins un groupe carboxy et deux dans le cas où $R_2$ = H et au moins un groupe aminé salifiable. L'invention se rapporte donc aussi aux sels des composés de formule générale (I) obtenus avec une base minérale ou organique thérapeutiquement compatible.

L'invention se rapporte également aux sels d'addition des composés de formule (I) obtenus avec un acide minéral ou organique thérapeutiquement compatible.

Les composés de formule (I) comportent au moins 3 atomes de carbone asymétrique. Selon la position des substituants et le degré d'hydrogénation, il existe 3 à 6 centres d'asymétrie. Les composés racémiques peuvent être dédoublés en leurs mélanges diastéréoisomères ou d'épimères, ou dédoublés en leurs énantiomères de manière connue. Ces divers isomères font partie de l'invention de même que les composés racémiques.

On préfère les composés correspondant à la formule (I) dans laquelle $R_3$ est un groupe alkyle linéaire ou ramifié de $C_3$ à $C_8$, cycloalkyl-alkyle de $C_4$ à $C_8$ ou un alkyle substitué $—CH_2—S—CHR_4R_5$ avec $R_4$ = H ou alkyle et $R_5$ = alcoxycarbonyle, les groupes alkyle et alcoxy ayant de 1 à 4 atomes de carbone. Par ailleurs $R_1$ peut être utilement un radical méthyle.

Les composés selon l'invention ainsi que leurs sels possèdent des propriétés pharmacologiques intéressantes. Ils exercent notamment une activité inhibitrice sur certaines enzymes, comme les carboxypolypeptidases, les enkephalinases ou la kininase II. Ils inhibent notamment la transformation du décapeptide angiotensine I en l'octapeptide angiotensine II, responsable dans certains cas de l'hypertension artérielle, en agissant sur l'enzyme de conversion.

L'emploi en thérapeutique de ces composés permet donc de réduire ou même supprimer l'activité de ces enzymes responsables de la maladie hypertensive ou de l'insuffisance cardiaque. L'action sur la kininase II a pour résultat l'augmentation de la bradykinine circulante et également la baisse de la tension artérielle par cette voie.

L'invention s'étend aussi aux compositions pharmaceutiques renfermant comme principe actif au moins un composé de formule générale I ou un de ses sels d'addition, avec une base ou un acide minéral ou organique, en association avec un excipient inerte, non toxique, pharmaceutiquement acceptable.

En vue de l'emploi en thérapeutique, les composés de formule générale I ou leurs sels sont présentés sous des formes pharmaceutiques convenant pour l'administration par voie intraveineuse ou buccale. Les compositions pharmaceutiques selon l'invention renferment, outre le principe actif, un ou plusieurs excipients inertes, non-toxiques convenant pour l'usage pharmaceutique et/ou un agent liant, un agent aromatisant, un agent de délitement, un agent édulcorant, un agent lubrifiant ou bien encore un véhicule liquide adapté à l'administration par voie intraveineuse.

Les compositions pharmaceutiques selon l'invention peuvent en outre contenir un autre principe

actif d'action synergique ou complémentaire.

Parmi ces derniers principes actifs, on pourra citer un diurétique et, notamment, un saliurétique, comme par exemple un thiazide, un dihydrothiazide, un chlorosulfamide, un acide dihydrobenzofuran 2-carboxylique ou un dérivé de l'acide phénoxy acétique. Des exemples de tels composés sont la N-(3'-Chloro 4'-sulfamyl benzamido)2-méthyl indoline, l'acide éthacrynique, le furosémide.

On pourra également ajouter des substances α-adrénolytiques comme le prazosin ou tout autre antihypertenseur.

La posologie utile peut varier largement en fonction de l'âge, du poids du patient, de la sévérité de l'indication thérapeutique ainsi que la voie d'administration. La voie d'administration préférée est la voie buccale mais la voie intraveineuse est également parfaitement appropriée au traitement de l'hypertension. D'une manière générale, la posologie unitaire s'échelonnera de préférence entre 5 et 100 mg.

L'invention comprend un procédé d'obtention des composés de formule générale I, selon lequel on soumet un ester d'alkyle d'acide perhydroindole di-carboxylique de formule générale II :

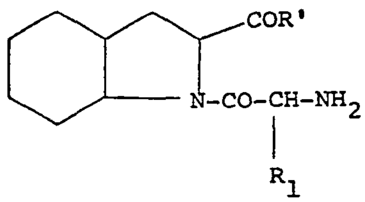

(II)

dans laquelle $R_1$ a la même signification que dans la formule I, et R' représente un radical hydroxy ou alcoxy inférieur, à une réaction d'alkylation réductive par un composé de formule générale III :

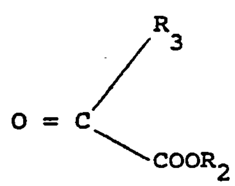

(III)

dans laquelle la définition des substituants $R_2$ et $R_3$ reste celle de la formule I, pour obtenir une amine de formule générale IV :

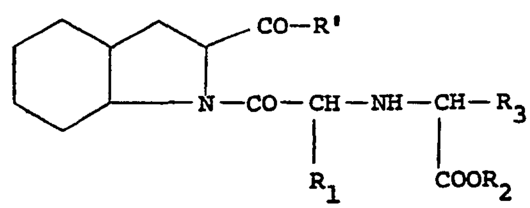

(IV)

dans laquelle R' possède la définition fournie précédemment pour la formule II et les symboles $R_1$, $R_2$ et $R_3$, gardent les significations fournies antérieurement, et, après alkylation réductive, ce composé intermédiaire obtenu est éventuellement soumis aux procédés de déprotection habituels tels que par exemple saponification totale ou partielle et/ou hydrogénolyse, et est ainsi transformé en composé de formule (I).

Les composés de formule générale II sont décrits ou peuvent être synthétisés selon la demande de brevet européen publiée sous le N° 0031741. L'alkylation réductive ci-dessus utilise le procédé décrit par R.F. BORCH, M.D. BERNSTEIN, et H. DUPONT DURST, JACS 93, 2897 (1971). On la réalise de préférence en milieu alcoolique et en présence d'un agent de déshydratation neutre et d'un cyano-borohydrure minéral ou organique. Les exemples suivants illustrent l'invention.

Exemple servant à illustrer le procédé de préparation selon l'invention mais ne conduisant pas à un composé de l'invention.

[N (Carboxy-1 éthyl) (S) alanyl]-2 (S) carboxy-3 tétrahydro-1,2,3,4 isoquinoléine.

Stade A

Acide tétrahydroisoquinoléine (lévogyre) 3-carboxylique.

Dans un ballon à trois tubulures surmonté d'un réfrigérant on introduit 15 g de (S) β-phénylalanine, puis 34 ml d'une solution de formol à 40 % et 105 ml d'acide chlorydrique concentré.

On chauffe pendant 30 mn au bain-marie bouillant. On obtient ainsi une solution claire, on laisse revenir le milieu réactionnel à température ambiante et on ajoute alors 15 ml de formol et 30 ml d'acide chlorhydrique concentré. On chauffe ensuite 3 heures au reflux du solvant. On laisse refroidir puis on sépare le précipité par filtration. Après essorage on le reprend par 200 ml d'eau bouillante et 400 ml d'éthanol chaud. On réunit les solutions qu'on neutralise par addition d'ammoniaque à 10 %.

L'acide tétrahydroisoquinoléine 3-carboxylique cristallique. On laisse reposer le mélange cristallin une nuit en glacière puis on sépare le précipité que l'on essore et lave à l'éthanol. On recueille ainsi 17,3 g de produit brut. Le produit est séché sous vide phosphorique.

Analyse $C_{10}H_{11}NO_2$ = 177

| | | | |
|---|---|---|---|
| Calculé | C % 67,78 | H % 6,26 | N % 7,90 |
| Trouvé | C % 66,87 | H % 6,20 | N % 7,96 |

Spectre IR

$NH_2^+$ Bande à 2 800-2 400 $cm^{-1}$
$COO^-$ Bande carbonyle à 1 630 $cm^{-1}$

Pouvoir rotatoire

$\alpha_D = -108°$ (c = 2,2 NaOH normal)

Stade B

Chlorydrate de tétrahydro-1,2,3,4 isoquinoléine (S) 3-carboxylate de méthyle.

Dans un ballon à trois tubulures on charge successivement 5 g d'acide tétrahydroisoquinoléine 3-carboxylique et 30 ml de méthanol. A cette suspension on ajoute par coulée tout en évitant que la température ne dépasse 0, + 5°, 6 g de chlorure de thionyle. L'addition dure environ 10 minutes. Après achèvement de cette addition, on maintient l'agitation pendant 2 heures à température ambiante, puis on porte au reflux du solvant 1 heure et demie. Une fois le mélange complètement dissous, on arrête le chauffage puis on évapore à sec. Le résidu est repris au méthanol à trois reprises et amené ensuite à sec. On recueille enfin 8 g de cristaux incolores que l'on purifie par trituration avec de l'éther. On sépare les cristaux par filtration, on les essore, on les lave à l'éther et on les sèche. On obtient ainsi 6,4 g de chlorhydrate de tétrahydroisoquinoléine 3-carboxylate de méthyle.

Analyse $C_{10}H_{13}NO_2$ ClH = 227,69

| | | | | |
|---|---|---|---|---|
| Calculé | C % 58,03 | H % 6,20 | N % 6,15 | Cl % 15,57 |
| Trouvé | C % 57,79 | H % 6,46 | N % 6,38 | Cl % 15,67 |

Spectre IR

Bande carbonyle à 1 735 $cm^{-1}$
Bande $NH_2^+$ à 2 800-2 400 $cm^{-1}$

Stade C

Terbutoxycarbonyl (S) alanyl-2 (S) méthoxycarbonyl-3 tétrahydro-1,2,3,4 isoquinoléine.

6,01 g (0,026 4 mol) de chlorhydrate préparé au stade précédent sont dissous dans 50 ml d'eau et la solution alcalinisée à pH 11 par $NH_4$ OH puis extraite par 2 fois 50 ml d'éther sulfurique. Les solutions éthérées réunies sont séchées sur sulfate de calcium, filtrées et évaporées à sec. L'amino ester résiduel (5,04 g) est dissous dans 30 ml de diméthylformamide et cette solution ajoutée à une solution agitée de 5 g (0,026 4 mol) de terbutylcarbonyl (S) alanine dans 30 ml de diméthylformamide refroidie à 0, + 5 °C. A la solution obtenue sont ajoutées successivement 3,6 g (0,026 4 mol) d'hydroxy-1 benztriazole dissous dans 40 ml de diméthylformamide puis 5,45 g (0,026 4 mol) de dicyclohexylcarbodiimide dissous dans 30 ml de chloroforme.

La masse réactionnelle est agitée pendant 18 h en laissant remonter à température ambiante. La dicyclohexylurée formée est filtrée et le filtrat évaporé à sec sous 0,1 mm Hg laisse un résidu qui est redissous dans 50 ml d'acétate d'éthyle et filtré à nouveau pour séparer un second jet de dicyclohexylurée. Le filtrat est lavé successivement par 80 ml de solution aqueuse saturée de NaCl, 2 × 40 ml de solution aqueuse d'acide citrique à 10 %, à nouveau 80 ml de solution aqueuse saturée de NaCl, 2 × 40 ml de solution aqueuse saturée de $CO_3HNa$, enfin par NaCl en solution aqueuse saturée jusqu'à neutralité.

La phase organique est séchée sur $SO_4Ca$, filtrée et évaporée à sec sous vide. Le résidu d'évaporation est le produit attendu :

Poids : 9,1 g (95 %)

Point de fusion : 98-100° (Kofler)

Analyse $C_{19}H_{26}N_2O_5$
Calculé C % 62,97 H % 7,23 N % 7,73
Trouvé C % 63,15 H % 7,05 N % 7,97

Stade D

Terbutoxycarbonyl (S) alanyl-2 (S) carboxy-3 tétrahydro-1,2,3,4 isoquinoléine.

1,45 g (0,004 mol) de composé préparé au stade précédent sont dissous dans 20 ml de méthanol et la solution obtenue additionnée de 4,4 ml (0,004 mol) de soude aqueuse normale.

La solution est abandonnée 20 heures à température ambiante. Le méthanol est évaporé sous vide de la trompe à eau et le résidu repris par 20 ml d'eau. Après extraction des insaponifiables par l'acétate d'éthyle, la phase aqueuse est acidifiée par 4,4 ml de HCl normal. Le précipité formé est extrait par 2 × 20 ml d'acétate d'éthyle qui est séché sur $SO_4$ Ca, filtré et évaporé. Le résidu obtenu est le produit cherché :
Poids : 1,3 g (93 %)
Analyse $C_{18}H_{24}N_2O_5$
Calculé C % 62,05 H % 6,94 N % 8,04
Trouvé C % 61,54 H % 6,93 N % 7,78

Stade E

(S) alanyl-2 (S) carboxy-3 tétrahydro-1,2,3,4 isoquinoléine.

1,1 g (0,003 16 mol) de dérivé préparé au stade précédent sont agités à + 5 °C avec 4,5 ml d'acide trifluoroacétique à l'abri de l'humidité.

La solution obtenue est concentrée à sec sous 0,1 mm Hg. Le résidu cristallin hygroscopique d'évaporation est le produit cherché, sous forme de trifluoroacétate solvaté par 0,5 mol d'acide trifluoroacétique :
Poids : 1,3 g (98 %)
Analyse $C_{32}H_{35}F_9N_4O_{12}$
Calculé C % 45,83 H % 4,21 N % 6,68
Trouvé C % 45,99 H % 4,62 N % 6,55

0,7 g (0,001 9 mol) de trifluoroacétate précédent sont transformés en 0,45 g (94 %) d'acide aminé libre correspondant, par passage sur 50 g de résine sulfonée (Dowex 50 W × 8 $H^+$) puis élution par 500 ml d'ammoniaque normal.
Point de fusion : 170 °C avec décomposition

Stade F

[N (carboxyl-1 éthyl) (S) alanyl]-2 (S) carboxy-3 tétrahydro-1,2,3,4 isoquinoléine. 0,849 g (0,003 4 mol) de (S) alanyl-2 carboxy-3 tétrahydro-1,2,3,4 isoquinoléine sont dissous en présence de 1,9 g (0,021 6 mol) d'acide pyruvique à 25 °C dans 22 ml de soude normale et 50 ml de tampon pH 7 prélevés sur une solution préparée à partir de 50 ml de solution 0,1 molaire de phosphate monosodique et 29,1 ml de soude décinormale. 0,45 g (0,007 2 mol) de cyanoborohydrure de sodium sont ajoutés en une fois. Le mélange réactionnel est abandonné 22 h à la température ambiante.

L'excès de cyanoborohydrure de sodium est décomposé par addition de 6 ml d'acide chlorhydrique concentré. La solution obtenue est passée sur résine échangeuse d'ion (Dowex 50 $H^+$). Après élution à l'eau distillée de la résine jusqu'à absence d'ion chlore, le produit fixé sur la résine est déplacé en éluant par 1 l de solution aqueuse normale d'ammoniaque. La solution ammoniacale est concentrée à sec sous vide de la trompe à eau. Le résidu d'évaporation est le sel monoammonique du produit cherché. Poids obtenu : 0,8 g (69,7 %).
Analyse $C_{16}H_{23}N_3O_5$
Calculé C % 56,96 H % 6,64 N % 12,95
Trouvé C % 57,79 H % 6,69 N % 12,70

Exemple 1

{N-[(RS) carboxy-1 éthyl)] (S) alanyl}-1 carboxy-2 perhydroindole.

Stade A

(RS) carboxy-2 indoline.

31,5 g de cette indoline (86 %) sont obtenus par saponification dans 250 ml de soude normale et 150 ml d'éthanol pendant 18 h à la température ambiante de 43 g (0,022 4 mol) d'ester éthylique

correspondant préparé selon E. J. COREY et al (J. Amer. Chem. Soc. 1970 *92*, p. 2476).

La solution hydroalcoolique est concentrée au 1/2, neutralisée par 25 ml d'acide chlorhydrique 10 N ; le précipité formé est filtré, lavé à l'eau et séché.

L'acide brut est purifié par passage sur une colonne de résine échangeuse d'ion (Dowex 50 W × 8 $H^+$) et élution par l'ammoniaque aqueuse 2 N. Le sel d'ammonium obtenu est dissous dans le minimum d'eau et l'acide précipité a la quantité théorique d'HCl. Il est essoré, lavé à l'eau et séché à l'air.

Analyse (du sel d'ammonium) $C_9H_{12}N_2O_2$

| | | | |
|---|---|---|---|
| Calculé | C % 59,99 | H % 6,71 | N % 15,54 |
| Trouvé | C % 60,22 | H % 6,71 | N % 15,06 |
| | 59,93 | 6,71 | 15,29 |

Stade B

(S) carboxy-2 indoline.

60,5 g (0,37 ml de (DL) carboxy-2 indoline préparé au Stade A sont ajoutés à une solution de 44,9 g (0,37 mol) de (+) α-méthyl-benzylamine dans 400 ml d'éthanol anhydre. Le précipité obtenu est essoré et digéré dans 350 ml d'isopropanol anhydre au reflux. Après refroidissement la suspension est filtrée, le précipité est lavé par un peu d'isopropanol et séché.

Poids obtenu de (L) carboxy-2 indoline, sel de (+) α-méthyl benzylamine 29,8 g.

$\alpha_D^{21} = 5{,}3°$ (C = 1 % éthanol).

La (S) carboxy-2 indoline est préparé avec un rendement théorique par dissolution de 10 g du sel précédent (0,029 mol) dans 50 ml d'eau et acidification par 29 ml d'acide chlorhydrique normal.

Le précipité est essoré, lavé à l'eau, distillé et séché. Pureté optique : 96 % (C.P.V. après dérivation sous forme d'amide de l'acide (−) camphanique).

La (R) carboxy-2 indoline a été obtenue par le même procédé à partir de (RS) carboxy indoline et de (−) α-méthyl benzylamine.

Les configurations absolues des acides (S) et (R) ont été déterminées, comme suit :

— Des quantités analytiques (environ 0,5 g) de chacun des acides sont transformés en esters éthyliques par traitement par le chlorure de thionyle et l'éthanol selon le procédé décrit au Stade C.

— Les esters sont réduits par l'hydrure de lithium aluminium selon E.J. COREY (loc. Cit) en alcools primaires correspondants, qui sont identifiés par leur pouvoir rotatoire aux alcools décrit par E.J. COREY dont les configurations absolues respectives sont connues.

Stade C

(S) éthoxycarbonyl-2 perhydroindole.

11 g de (L) carboxy-2 indoline, sel de (+) α-méthyl benzylamine (0,032 mol) préparé au Stade B sont dissous dans 100 ml d'eau et transformés en acide correspondant par addition de 32 ml d'HCl N. L'acide est essoré, lavé à l'eau et séché en dessicateur sur anhydride phosphorique, puis mis en suspension dans 50 ml d'éthanol anhydre. A 0, + 5°, 3,9 ml de chlorure de thionyle sont ajoutés en 10 minutes sous agitation et l'agitation est maintenue 1 heure à 25 °C puis 1 heure à 50 °C.

Le mélange est abandonné la nuit à 25°, puis concentré à sec sous vide de la trompe à eau à 40° et repris par 50 ml de benzène anhydre et essoré.

Le chlorhydrate de (S) éthoxycarbonyl-2 indoline obtenu est hydrogéné en solution dans 150 ml d'eau en présence de 2 g de charbon palladié pendant 8 heures à 45 °C sous 50 kg/cm².

Après refroidissement et filtration du catalyseur, le filtrat est évaporé à sec. Le résidu est le produit cherché sous forme de chlorhydrate.

Poids : 6,9 g (93 %)

Analyse $C_{11}H_{20}Cl\ NO_2$

| | | | | |
|---|---|---|---|---|
| Calculé | C % 56,52 | H % 8,62 | N % 5,99 | Cl % 15,17 |
| Trouvé | C % 55,52 | H % 8,53 | N % 5,96 | Cl % 15,16 |

Stade D

N [(S) t-Boc alanyl] (S) éthoxycarbonyl-2 perhydroindole.

Une solution comprenant 3 g (0,012 8 mol) de chlorhydrate de (S) éthoxycarbonyl-2 perhydroindole préparé au stade précédent (C), 15 ml de diméthylformamide (D.M.F.) séché et 1,8 ml de triéthylamine, est ajoutée à une solution refroidie à + 5 °C et agitée de 2,42 g (0,012 8 mol) de t-Boc (L) alanine dans 15 ml de D.M.F. Au mélange obtenu sont additionnées successivement une solution de 1,7 g (0,012 8 mol) de N-hydroxy benz-triazole dans 20 ml de D.M.F. puis une solution de 2,64 g (0,012 8 mol) de dicyclohexyl carbodiimide dans 15 ml de chloroforme sec.

Après 65 heures d'agitation à 25°, la dicyclohexylurée formée est filtrée et lavée à l'acétate d'éthyle.

Les filtrats réunis sont lavés successivement par 80 ml de solution aqueuse saturée de NaCl, 2 fois 40 ml de solution concentrée d'acide citrique, 2 fois 40 ml de solution aqueuse saturée de $CO_3H$ Na, puis à nouveau 2 fois 40 ml de solution de NaCl.

La solution organique est séchée sur $SO_4Ca$, filtrée, concentrée à sec sous vide de la trompe à eau, le résidu est repris par 100 ml d'acétate d'éthyle. La solution est filtrée pour éliminer les dernières traces de dicyclohexylurée, et le filtrat concentré à sec laisse un résidu qui est le produit cherché, sous forme d'une huile très visqueuse.

Poids : 3,8 g (81 %)
Analyse $C_{19}H_{32}N_2O_5$
Calculé C % 61,93 H % 8,75 N % 7,60
Trouvé C % 61,76 H % 8,56 N % 7,77

Stade E

N [(S) t-Boc alanyl] (S) carboxy-2 perhydroindole

3,6 g (0,009 8 mol) d'ester obtenu au Stade D sont dissous dans 30 ml de méthanol en présence de 11 ml de solution aqueuse normale de soude.

Après 20 heures à 25°, le méthanol est évaporé sous vide de la trompe à eau et 60 ml d'eau sont ajoutés. La solution est lavée par 2 fois 50 ml d'acétate d'éthyle pour éliminer les insaponifiables puis acidifiée par 11 ml d'acide chlorhydrique N. Le précipité blanc formé est extrait par 2 × 50 ml d'acétate d'éthyle, qui sont réunis et lavés à l'eau, séchés sur $SO_4Ca$, filtrés et concentrés à sec. Le résidu est le produit cherché.

Poids : 1,9 g (57 %)
Analyse $C_{17}H_{28}N_2O_5$
Calculé C % 59,98 H % 8,29 N % 8,23
Trouvé C % 59,10 H % 8,16 N % 7,81

Stade F

(S) alanyl-1 (S) carboxy-2 perhydroindole

1,6 g (0,004 7 mol) d'acide préparé au stade précédent (E) sont agités à 0, + 5 °C en solution dans 10 ml d'acide trifluoroacétique pendant 1 heure, puis pendant 15 minutes supplémentaires à température ambiante.

Après évaporation à sec sous vide de la pompe à palettes, le résidu dissous dans 15 ml d'eau est passé sur une colonne de résine échangeuse d'ion (Dowex W + 8 $H^+$). La colonne est éluée par 1 l d'ammoniaque aqueuse 2 N. Les éluats sont concentrés à sec sous vide. Le résidu obtenu est le produit cherché.

Poids : 0,90 g (95 %)
Analyse $C_{12}H_{20}N_2O_3$
Calculé C % 59,98 H % 8,39 N % 11,10
Trouvé C % 58,53 H % 8,24 N % 11,43

Stade G

{N [(RS) carboxy-1 éthyl] (S) alanyl}-1 (S) carboxy-2 perhydroindole.

0,7 g (0,002 91 mol) de N (S) alanyl (S) carboxy-2 perhydroindole préparé au stade précédent (F) et 1,67 g (0,018 3 mol) d'acide pyruvique sont dissous dans 18 ml de soude aqueuse normale et 40 ml de tampon pH 7, la solution obtenue est soumise à la réduction par 0,400 g (0,006 4 mol) de cyanoborohydrure de sodium comme décrit dans l'exemple d'illustration stade F.

Après traitement par l'acide chlorhydrique concentré et passage sur résine échangeuse d'ion (Dowex 50 $H^+$), l'éluat ammoniacal final évaporé à sec, laisse 0,76 g (79 %) de résidu qui est le produit cherché sous forme de sel monoammonique.

Analyse $C_{15}H_{27}N_3O_5$
Calculé C % 54,70 H % 8,26 N % 12,76
Trouvé C % 54,10 H % 7,78 N % 12,77

Exemple 2

N-[{(R,S) éthoxycarbonyl-1 ethylthio}-2 (RS) éthoxycarbonyl-1 ethyl] (S) alanyl-1 (S) carboxy-2 perhydroindole.

1 g (4,17 m mole) de (S) alanyl-1 (S) carboxy-2 perhydroindole, préparé comme décrit dans l'exemple

1 stade F et 4,72 g (19 m mole de (RS) éthoxycarbonyl-1 ethylthio pyruvate d'éthyle sont dissous dans 50 ml d'éthanol anydre en présence de 15 g de tamis moléculaire 4 Å. Après 45 minutes d'agitation à température ambiante, 0,25 g de Cyanoborohydrure de sodium en solution dans 2,25 ml d'éthanol anhydre sont ajoutés en 6 heures.

Après séparation du tamis moléculaire par filtration, le filtrat est concentré à sec sous pression réduite et le résidu dissous dans 100 ml d'éther sulfurique. La solution est extraite par 2 × 100 ml d'eau distillée, puis séchée sur sulfate de calcium, filtrée et chromatographiée sur 200 g de silice (Merck F 254) en éluant par un mélange chlorure de méthylène/méthanol 180/20. 0,5 g (25 %) de produit cherché sont obtenus sous forme de sel sodique.

Analyse $C_{22}H_{35}N_2NaO_7S$

Calculé C % 53,43 H % 7,13 N % 5,66 S % 6,48

Trouvé C % 53,28 H % 7,09 N % 5,19 S % 5,92

Le [(RS) éthoxycarbonyl-1 ethylthio] pyruvate d'éthyle intermédiaire est préparé par condensation du bromopyruvate d'éthyle avec le (RS) thiolactate d'éthyle en présence de pyridine selon le procédé décrit pour des dérivés voisins dans le J. of Heter. Chem. (1973) 10/4 p. 679-681.

$E_{15}$ = 165-170

Rdt 67 %

## Exemple 3

N-[(ethoxycarbonylméthylthio)-2 (RS) ethoxycarbonyl-1 éthyl]-(S)-alanyl-1 (S) carboxy-2 perhydroindole.

Préparé comme dans l'exemple 2 à partir de 1 g (4,17 m mole) de (S) alanyl-1 (S) carboxy-2 perhydro indole, 4,45 (1,9 mole) de carbethoxy methyl thio pyruvate d'éthyle et 0,25 g de cyanoborohydrure de sodium.

Après purification par chromatographie, on obtient 0,26 g (14 %) de produit cherché.

Analyse $C_{21}H_{34}N_2O_7S$

Calculé C % 55,00 H % 7,47 N % 6,11 S % 6,99

Trouvé C % 54,71 H % 7,32 N % 5,94 S % 7,01

Le carbéthoxy méthyl mercapto pyruvate d'éthyl intermédiaire est préparé par condensation dubromopyruvate d'éthyle avec le thioglycolate d'éthyl selon le procédé décrit par la référence cité dans l'exemple 2. $E_{15}$ = 165-175 Rdt 50 %.

Les composés préparés dans les exemples précédents ainsi que d'autres composés de formule (I) préparés de manière semblable ont été rassemblés dans le tableau suivant. Par commodité, les symboles A et n ne sont mentionnés que pour les valeurs A = cycle benzénique et n = 1. Pour tous les autres composés A signifie cycle saturé et n = 0 (perhydroindole de formule I').

Le tableau donne les valeurs caractéristiques des composés en infra-rouge (IR) et résonance magnétique nucléaire (RMN) :

s est pour singulet,
d est pour doublet,
q est pour quadruplet,
m pour multiplet.

(Voir tableau pages suivantes)

Tableau

| Comp. N° | $R_1$ | $R_2$ | $R_3$ | FORME (sel) |
|---|---|---|---|---|
| 1 Ex.1 | $CH_3$ | H | $CH_3$ | sel d'amonium |
| 2 | $CH_3$ | $C_2H_5$ | $-CH_2-S-CH$ 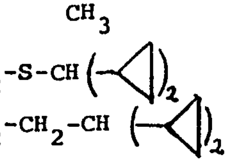 | ———— |
| 3 | $CH_3$ | $C_2H_5$ | $-CH_2-CH_2-CH$ | maléate acide |
| 4 | $CH_3$ | $C_2H_5$ | $CH_3$ | maléate acide |
| 5 | $CH_3$ | $C_2H_5$ | $-CH_2-S-CH$ 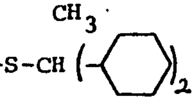 | sel sodique |
| 6 Ex.2 | $CH_3$ | $C_2H_5$ | (RS)<br>$-CH_2-S-CH-COOC_2H_5$<br>$CH_3$ | sel sodique |
| 7 | $CH_3$ | $C_2H_5$ | (S)<br>$-CH_2-S-CH-COOC_2H_5$<br>$CH_3$ | maléate acide |
| 8 | $CH_3$ | $C_2H_5$ | $-CH_2-CH(CH_3)_2$ | sel sodique |

Tableau (Suite)

| Comp. N° | $R_1$ | $R_2$ | $R_3$ | FORME (sel) |
|---|---|---|---|---|
| 9 Ex.3 | $CH_3$ | $C_2H_5$ | $-CH_2-S-CH_2-COOC_2H_5$ | —— |
| 10 | $CH_3$ | $C_2H_5$ | $n-C_4H_9$ | sel sodique |
| 11 | $CH_3$ | $C_2H_5$ | $n-C_3H_7$ | —— |
| 12 | $CH_3$ | $C_2H_5$ | $-CH_2-$ 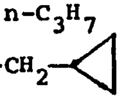 | sel sodique |
| 13 | $CH_3$ | $C_2H_5$ | $i-C_3H_7$ | sel sodique |
| 14 | $CH_3$ | $C_2H_5$ | $C_2H_5$ | sel sodique |
| 15 | $CH_3$ | $C_2H_5$ | $n-C_5H_{11}$ | sel sodique |
| 16 | $CH_3$ | $C_2H_5$ | $n-C_6H_{13}$ | —— |
| 17 | $CH_3$ | $C_2H_5$ | $n-C_8H_{17}$ | trifluoroacétate |

Tableau (Suite 2)

| Comp. | I.R. ( $\nu_s$ en $cm^{-1}$) | R.M.N. dans $CDCl_3$ : déplacements chimiques (ppm)/TMS |
|---|---|---|
| 1 | NH : 3500-2500<br>C=O : 1600 | m. : 3H(4,8-4) Massifs:18H(2,5-1,3)<br>2H(6,35) |
| 2 | | Massifs:17H(1,6-0,8) 6H(4,5-3,5) 2H(5,7-5,2)<br>10H(0,7-0,1) 3H(3,2-1,9) |
| 3 | | Massifs:21H(2,7-1) 6H(4,6-3,7) s. : 2H(6,4)<br>11H(0,8-0,1) 4H(11,2) |
| 4 | | Massifs:20H(2,7-1,1) 4H(10,3) s. : 2H(6,4)<br>6H(4,7-3,9) |
| 5 | NH3700-3200 CO ester 1730<br>CO amide 1650-1600<br>1690<br>COamide 1650-1600 | Massifs: 8H(4,7-3,2) d. : 2H (2,9)<br>39H(2,5-1) |
| 6 | NH3300 CO ester 1725<br>CO amide 1620 | Massifs:11H(4,5-2,6) s. : 2H(6,5) 4H échangeables (11,1)<br>23H(2,5-1) |
| 7 | | Massifs: 9H(4,7-3,2)<br>25H(2,5-1) |
| 8 | NH3600-2300 COester 1725<br>COamide 1630 | Massifs: 6H(3-4,5) d. : 6H(1)<br>20H(1,2-2,5) |

Tableau (Suite 3)

| Comp | I.R. ( $\nu_s$ en $cm^{-1}$ ) | R.M.N. dans $CDCl_3$ : déplacements chimiques (ppm)/TMS |
|---|---|---|
| 9 | NH 3700-2500 C=O ester 1720<br>C=O amide 1625 | Massifs: 18H(2-1) 2H(2,5-2) q. : 4H(4,25) s. : 2H (3,4)<br>4H(4,5-3,2) d. : 2H(3) 2H échangeables |
| 10 | NH 3600-3100 C=O ester 1725<br>C=O amide 1620 | Massifs: 6H(3-4,5) 27H(0,1 -2,5) |
| 11 | NH 3300 C=O ester 1725<br>C=O amide 1620 | Massifs: 24H(2,4-0,7) s. 2H(6,8)<br>6H(4,6-3,4) |
| 12 | NH 3300 C=O ester 1725<br>C=O amide 1610 | Massifs: 25H(2,5-0)<br>6H(4,5-3) |
| 13 | NH 3300 C=O ester 1725<br>C=O amide | Massifs: 5H(4,5-3) 25H(0,7-2,5)<br>1H(2,9) |
| 14 | NH 3600-2500 C=O ester 1730<br>C=O amide 1610 | Massifs: 6H(3-4,6)<br>23H(0,6-2,5) |
| 15 | NH 3300 C=O ester 1725<br>C=O amide 1610 | Massifs: 7H(3-5)<br>28H(0,5-2,6) |
| 16 | $NH_2^+$ 3600-2400 C=O ester 1730<br>C=O amide 1650-1550 | Massifs: 6H(3-4,7) 2H échangeables (5,9)<br>30H (0,8-2,6) |
| 17 | $NH_2^+$ 3500-2300 C=O ester 1740<br>C=O amide 1650 | Massifs: 6H(3,5-4,6) 3H échangeables (8-9)<br>34H(0,6-2,7) |

Etude pharmacologique des composés de l'invention.

Les composés selon l'invention ont été testés par l'administration i.v ou p.o chez le chien éveillé.

La pression artérielle des chiens a été mesurée par un capteur de pression (Statham P 23 Db) après cathétérisation de l'aorte par l'intermédiaire de l'artère fémorale. L'enregistrement est réalisé par un appareil enregistreur (Brush 400).

L'angiotensine I et l'angiotensine II sont injectées aux animaux par voie intra-veineuse à la dose de 0,3 $\gamma$/kg. On administre ensuite les composés selon l'invention par voie buccale ou intraveineuse à la dose de 1 à 5 mg/kg.

On constate une inhibition de l'activité hypertensive de l'angiotensine I allant de 50 à 100 % 30 à 90 minutes après l'administration et se maintenant de 40 à 80 % à plus de 6 heures après l'administration. Certains composés restent actifs après 24 heures, ce qui n'est le cas d'aucun composé connu jusqu'à présent (en particulier le captopril qui est le seul composé commercial). Par ailleurs, les composés de l'invention ne semblent avoir aucune toxicité ($DL_O > 500$ mg/kg i.p. chez la souris).

Exemple de formulation

| | |
|---|---|
| {N-[((S) éthoxycarbonyl-1 éthylthio)-2 (R,S) éthoxycarbonyl-1 éthyl] (S) alanyl}-1 (S) carboxy-2 perhydroindole (maléate) | 10 mg |
| amidon de blé | 120 mg |
| amidon de maïs | 115 mg |
| caséine formolé | 20 mg |
| stéarate de magnésium | 15 mg |
| talc | 20 mg |

pour 1 comprimé.

**Revendications** (pour les Etats contractants : BE, CH, DE, FR, GB, IT, LI, LU, NL, SE)

1. Composés répondant à la formule générale :

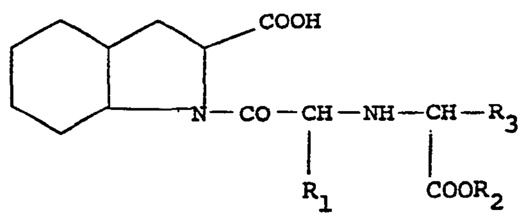

(I)

dans laquelle

$R_1$ représente un groupe alkyle inférieur de 1 à 4 atomes de carbone,

$R_2$ représente un atome d'hydrogène ou un groupe alkyle de 1 à 4 atomes de carbone,

$R_3$ représente un groupe alkyle linéaire ou ramifié, ou mono- ou di-cycloalkyl-alkyle, chacun de ces groupes ayant au plus et au total 9 atomes de carbone, ou bien un groupe alkyle substitué de formule

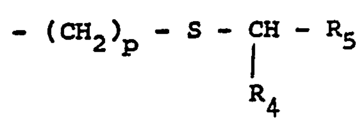

avec

$R_4$ = H, alkyle inférieur ($C_1$ à $C_4$) ou cycloalkyle (de $C_3$ à $C_6$)

$R_5$ = H, alkyle inférieur ($C_1$ à $C_4$), cycloalkyle ($C_3$ à $C_6$) ou alcoxycarbonyle, et

p = 1 ou 2, sous leur forme racémique ou d'isomères optiques, et leurs sels obtenus avec une base minérale ou organique thérapeutiquement compatible, ou leurs sels d'addition obtenus avec un acide minéral ou organique pharmaceutiquement compatible.

2. Composés selon la revendication 1, répondant à la formule (I) dans laquelle $R_3$ est un groupe alkyle linéaire ou ramifié de $C_3$ à $C_8$, cycloalkyl-alkyle de $C_4$ à $C_8$ ou un alkyle substitué de formule :

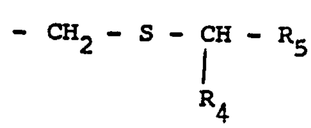

avec $R_4$ = H ou alkyle de $C_1$ à $C_4$ et $R_5$ = alcoxy ($C_1$ à $C_4$) carbonyle.

3. Composés selon la revendication 2, répondant à la formule (I) dans laquelle $R_1$ est un radical méthyle.

4. Le {N-[((RS) éthoxycarbonyl-1 éthylthio)-2 (RS) éthoxycarbonyl-1 éthyl] (S) alanyl}-1 (S) carboxy-2 perhydroindole, ses isomères (S), ainsi que leur maléate.

5. Le {N-[(R,S) éthoxycarbonyl-1 méthyl-3 butyl] (S) alanyl}-1 (S) carboxy-2 perhydroindole, son isomère (S) et leur sel de sodium.

6. Le {N-[(R,S) éthoxycarbonyl-1 pentyl] (S) alanyl}-1 (S) carboxy-2-perhydroindole, son isomère (S) et leur sel de sodium.

7. Le {N-[(R,S) éthoxycarbonyl-1 butyl] (S)-alanyl}-1 (S) carboxy-2 perhydroindole, son isomère (S) et leur sel de sodium.

8. Le {N-[(R,S) éthoxycarbonyl-1 cyclopropyl-2 éthyl]-(S) alanyl-1 (S) carboxy-2 perhydroindole, son isomère (S) et leur sel de sodium.

9. Procédé de préparation des composés selon la revendication 1, procédé caractérisé en ce que l'on soumet un ester d'alkyle d'acide di-carboxylique de formule générale II :

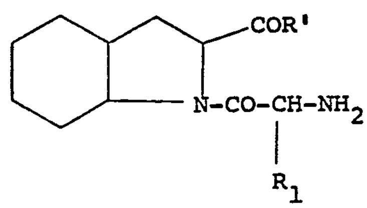

(II)

dans laquelle $R_1$ a la même signification que dans la formule I, et R' représente un radical hydroxy ou alcoxy inférieur, à une réaction d'alkylation réductive par un composé de formule générale III :

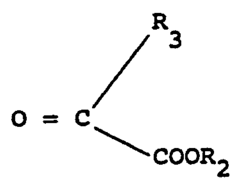

(III)

dans laquelle la définition de substituants $R_2$ et $R_3$ est la même que dans la revendication 1, pour obtenir une amine de formule générale IV :

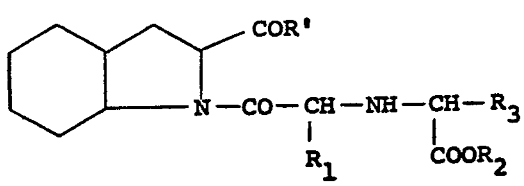

(IV)

dans laquelle R' possède la définition fournie précédemment pour la formule II et les symboles $R_1$, $R_2$ et $R_3$, gardent les significations fournies antérieurement, et après alkylation réductive, ce composé intermédiaire obtenu est éventuellement soumis aux procédés de déprotection habituels tels que par exemple saponification totale ou partielle et/ou hydrogénolyse, et est ainsi transformé en composé de formule (I).

10. Composition pharmaceutique renfermant à titre de principe actif au moins un composé selon l'une quelconque des revendications 1 à 8, en association avec un excipient ou un véhicule inerte non-toxique thérapeutiquement compatible.

**Revendication** (pour l'Etat contractant AT)

Procédé de préparation de composés répondant à la formule générale :

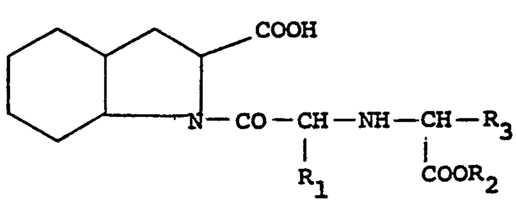

(I)

dans laquelle

$R_1$ représente un groupe alkyle inférieur de 1 à 4 atomes de carbone,

$R_2$ représente un atome d'hydrogène ou un groupe alkyle de 1 à 4 atomes de carbone,

$R_3$ représente un groupe alkyle linéaire ou ramifié, ou mono- ou di-cycloalkyl-alkyle, chacun de ces groupes ayant au plus et au total 9 atomes de carbone, ou bien un groupe alkyle substitué de formule :

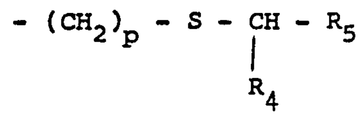

avec

$R_4$ = H, alkyle inférieur ($C_1$ à $C_4$) ou cycloalkyle (de $C_3$ à $C_6$)

$R_5$ = H, alkyle inférieur ($C_1$ à $C_4$), cycloalkyle ($C_3$ à $C_6$) ou alcoxycarbonyle, et p = 1 ou 2, sous leur forme racémique ou d'isomères optiques, et leurs sels obtenus avec une base minérale ou organique thérapeutiquement compatible, ou leurs sels d'addition obtenus avec un acide minéral ou organique pharmaceutiquement compatible, procédé caractérisé en ce que l'on soumet un ester d'alkyle d'acide di-carboxylique de formule générale II :

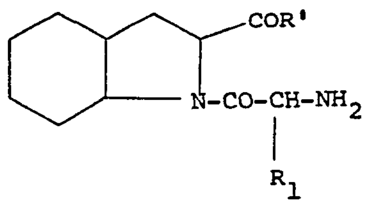

(II)

dans laquelle $R_1$ a la même signification que dans la formule I, et R' représente un radical hydroxy ou alcoxy inférieur, à une réaction d'alkylation réductive par un composé de formule générale III :

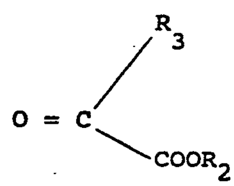

(III)

dans laquelle la définition de substituants $R_2$ et $R_3$ est la même que dans la revendication 1, pour obtenir une amine de formule générale IV :

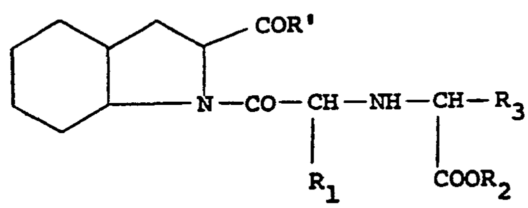

(IV)

dans laquelle R' possède la définition fournie précédemment pour la formule II et les symboles $R_1$, $R_2$ et $R_3$, gardent les significations fournies antérieurement, et après alkylation réductive, ce composé intermédiaire obtenu est éventuellement soumis aux procédés de déprotection habituels tels que par exemple saponification totale ou partielle et/ou hydrogénolyse, et est ainsi transformé en composé de formule (I).

**Claims** (for the Contracting States : BE, CH, DE, FR, GB, IT, LI, LU, NL, SE)

1. Compounds corresponding to the general formula :

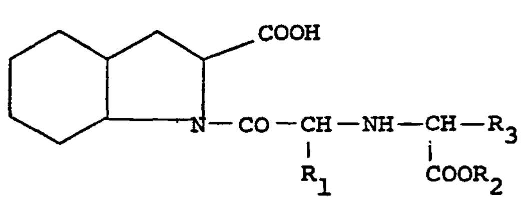

(I)

in which

$R_1$ represents a lower alkyl group having from 1 to 4 carbon atoms,

$R_2$ represents a hydrogen atom or an alkyl group having from 1 to 4 carbon atoms,

$R_3$ represents a linear or branched alkyl group, or a mono- or di-cycloalkyl-alkyl group, each of these groups having a maximum of 9 carbon atoms in total, or a substituted alkyl group of the formula :

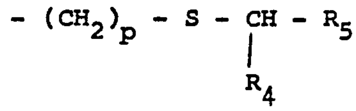

in which

$R_4$ = H, ($C_1$-$C_4$)-lower alkyl or ($C_3$-$C_6$)-cycloalkyl,

$R_5$ = H, ($C_1$-$C_4$)-lower alkyl, ($C_3$-$C_6$)-cycloalkyl or alkoxycarbonyl, and

p = 1 or 2, in their racemic form or in the form of their optical isomers, and their salts obtained with a therapeutically compatible mineral or organic base, or their addition salts obtained with a pharmaceutically compatible mineral or organic acid.

2. Compounds according to claim 1, corresponding to formula (I), in which $R_3$ is a linear or branched ($C_3$-$C_8$)-alkyl group, a ($C_4$-$C_8$)-cycloalkylalkyl or a substituted alkyl of the formula :

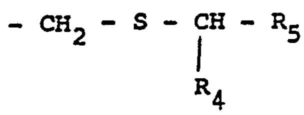

in which $R_4$ = H or ($C_1$-$C_4$)-alkyl and $R_5$ = (($C_1$-$C_4$)-alkoxy)-carbonyl.

3. Compounds according to claim 2, corresponding to the formula (I) in which $R_1$ is a methyl radical.

4. 1-{N-[2-(1-(RS)-ethoxycarbonylethylthio)-1-(RS)-ethoxycarbonylethyl]-(S)-alanyl}-2-(S)-carboxy-perhydroindole, its (S)-isomers and their maleate.

5. 1-{N-[1-(R,S)-ethoxycarbonyl-3-methylbutyl]-(S)-alanyl}-2-(S)-carboxy-perhydroindole, its (S)-isomer and their sodium salt.

6. 1-{N-[1-(R,S)-ethoxycarbonylpentyl]-(S)-alanyl}-2-(S)-carboxy-perhydroindole, its (S)-isomer and their sodium salt.

7. 1-{N-[1-(R,S)-ethoxycarbonylbutyl]-(S)-alanyl}-2-(S)-carboxy-perhydroindole, its (S)-isomer and their sodium salt.

8. 1-{N-[1-(R,S)-ethoxycarbonyl-2-cyclopropylethyl]-(S)-alanyl}-2-(S)-carboxy-perhydroindole, its (S)-isomer and their sodium salt.

9. Process for the preparation of the compounds according to claim 1, characterised in that a dicarboxylic acid alkyl ester of the general formula II :

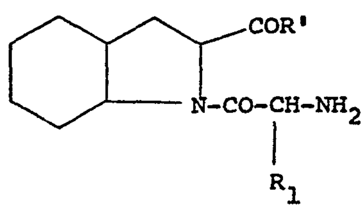

(II)

in which $R_1$ has the same meaning as in formula I, and R' represents a hydroxy radical or a lower alkoxy radical, is subjected to a reductive alkylation reaction with a compound of the general formula III :

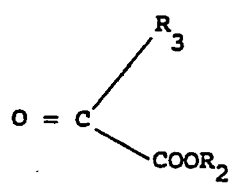

(III)

in which the definition of the substituents $R_2$ and $R_3$ is the same as in claim 1, to obtain an amine of the general formula IV :

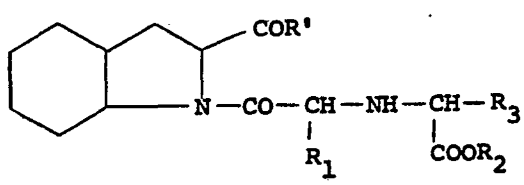

(IV)

in which R' has the meaning given above for formula II and the symbols $R_1$, $R_2$ and $R_3$ retain the meanings given previously, and, after reductive alkylation, this intermediate compound obtained is optionally subjected to customary deprotection processes, such as, for example, total or partial hydrolysis and/or hydrogenolysis, and is thus converted into a compound of the formula (I).

10. Pharmaceutical composition containing as active ingredient at least one compound according to any one of claims 1 to 8, in conjunction with an excipient or a therapeutically compatible non-toxic inert carrier.

**Claim** (for the Contracting State AT)

Process for the preparation of compounds corresponding to the general formula :

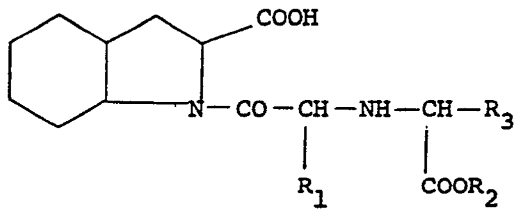

(I)

in which

$R_1$ represents a lower alkyl group having from 1 to 4 carbon atoms,

$R_2$ represents a hydrogen atom or an alkyl group having from 1 to 4 carbon atoms,

$R_3$ represents a linear or branched alkyl group, or a mono- or di-cycloalkyl-alkyl group, each of these groups having a maximum of 9 carbon atoms in total, or a substituted alkyl group of the formula :

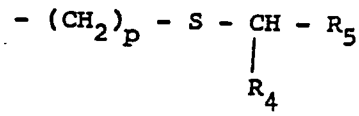

in which

$R_4$ = H, ($C_1$-$C_4$)-lower alkyl or ($C_3$-$C_6$)-cycloalkyl

$R_5$ = H, ($C_1$-$C_4$)-lower alkyl, ($C_3$-$C_6$)-cycloalkyl or alkoxycarbonyl, and p = 1 or 2, in their racemic form or in the form of their optical isomers, and their salts obtained with a therapeutically compatible mineral or organic base, or their addition salts obtained with a pharmaceutically compatible mineral or organic acid, which process is characterised in that a dicarboxylic acid alkyl ester of the general formula II :

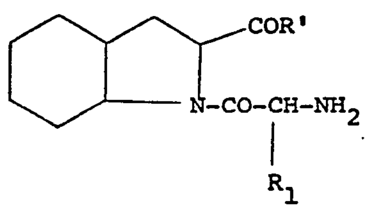

(II)

in which $R_1$ has the same meaning as in formula I, and R' represents a hydroxy radical or a lower alkoxy radical, is subjected to a reductive alkylation reaction with a compound of the general formula III :

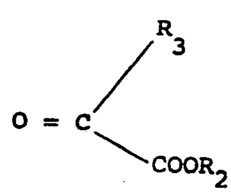

(III)

in which the definition of the substituents $R_2$ and $R_3$ is the same as in claim 1, to obtain an amine of the general formula IV :

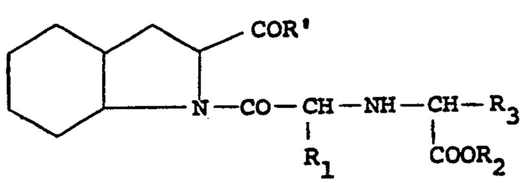

(IV)

in which R′ has the meaning given above for formula II and the symbols $R_1$, $R_2$ and $R_3$ retain the meanings given previously, and, after reductive alkylation, this intermediate compound obtained is optionally subjected to customary deprotection processes, such as, for example, total or partial hydrolysis and/or hydrogenolysis, and is thus converted into a compound of the formula (I).

**Ansprüche** (für die Vertragsstaaten : BE, CH, DE, FR, GB, IT, LI, LU, NL, SE)

1. Verbindungen der allgemeinen Formel :

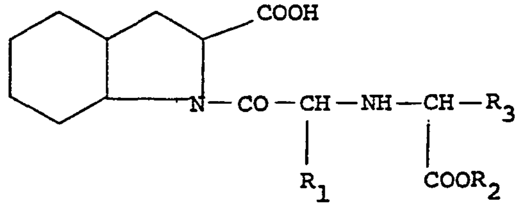

(I)

in der

$R_1$ eine Niedrigalkylgruppe mit 1 bis 4 Kohlenstoffatomen,

$R_2$ ein Wasserstoffatom oder eine Alkylgruppe mit 1 bis 4 Kohlenstoffatomen,

$R_3$ eine geradkettige oder verzweigte Alkylgruppe oder eine Mono- oder Dicycloalkylalkylgruppe, wobei diese Gruppen jeweils höchstens und insgesamt 9 Kohlenstoffatome aufweisen, oder eine substituierte Alkylgruppe der Formel :

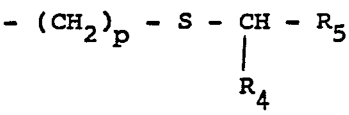

worin

$R_4$ ein Wasserstoffatom, eine Niedrigalkylgruppe ($C_1$-$C_4$) oder eine Cycloalkylgruppe ($C_3$-$C_6$),

$R_5$ ein Wasserstoffatom, eine Niedrigalkylgruppe ($C_1$-$C_4$), eine Cycloalkylgruppe ($C_3$-$C_6$) oder eine Alkoxycarbonylgruppe und

p 1 oder 2 darstellen, bedeuten, in Form des Racemats oder der optischen Isomeren und deren Salze mit einer anorganischen oder organischen therapeutisch verträglichen Base oder deren Additionssalze mit einer anorganischen oder organischen pharmazeutisch verträglichen Säure.

2. Verbindungen nach Anspruch 1 der Formel (I), in der $R_3$ eine geradkettige oder verzweigte Alkylgruppe ($C_3$-$C_8$), eine Cycloalkylalkylgruppe ($C_4$-$C_8$) oder eine substituierte Alkylgruppe der Formel :

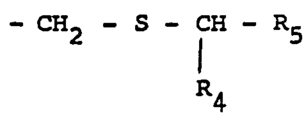

in der $R_4$ ein Wasserstoffatom oder eine Alkylgruppe ($C_1$-$C_4$) und $R_5$ eine Alkoxy ($C_1$-$C_4$)-carbonylgruppe darstellen, bedeuten.

3. Verbindungen nach Anspruch 2 der Formel (I), in der $R_1$ eine Methylgruppe bedeutet.

4. 1(S)-{N-[2-((R,S)-1-Ethoxycarbonyl-ethylthio)-(R,S)-1-ethoxycarbonyl-ethyl]-(S)-alanyl}-2-carboxy-perhydroindol, dessen (S)-Isomeren und deren Maleat.

5. 1(S)-{N-[(R,S)-1-Ethoxycarbonyl-3-methyl-butyl]-(S)-alanyl}-2-carboxy-perhydroindol, dessen (S)-Isomeres und deren Natriumsalz.

6. 1(S)-{N-[(R,S)-1-Ethoxycarbonyl-pentyl]-(S)-alanyl}-2-carboxy-perhydroindol, dessen (S)-Isomeres und deren Natriumsalz.

7. 1(S)-{N-[(R,S)-1-Ethoxycarbonyl-butyl]-(S)-alanyl}-2-carboxy-perhydroindol, dessen (S)-Isomeres und deren Natriumsalz.

8. 1(S)-{N-[(R,S)-1-Ethoxycarbonyl-2-cyclopropyl-ethyl]-(S)-alanyl}-2-carboxy-perhydroindol, dessen (S)-Isomeres und deren Natriumsalz.

9. Verfahren zur Herstellung der Verbindungen nach Anspruch 1, dadurch gekennzeichnet, daß man einen Alkylester der Dicarbonsäure der allgemeinen Formel (II) :

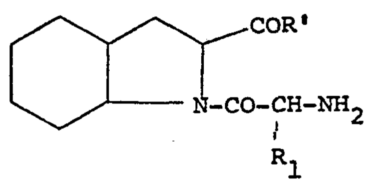

(II)

in der $R_1$ die bezüglich der allgemeinen Formel I angegebenen Bedeutungen besitzt und R' eine Hydroxylgruppe oder eine Niedrigalkoxygruppe darstellt, einer reduktiven Alkylierungsreaktion mit einer Verbindung der allgemeinen Formel III :

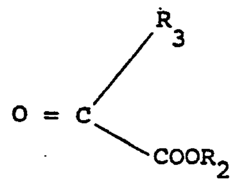

(III)

worin $R_2$ und $R_3$ die Anspruch 1 angegebenen Bedeutungen besitzen, zur Bildung eines Amins der allgemeinen Formel IV unterwirft :

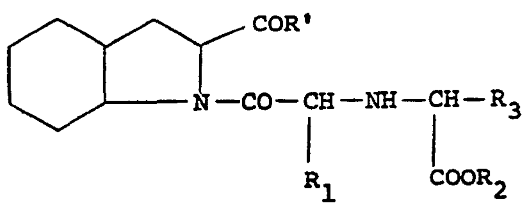

(IV)

in der R' die oben bezüglich der Formel II angegebenen Bedeutungen besitzt und $R_1$, $R_2$ und $R_3$ die obigen Bedeutungen beibehalten, und nach der reduktiven Alkylierung die erhaltene Zwischenverbindung gegebenenfalls üblichen Verfahren zur Schutzgruppenabspaltung, wie beispielsweise einer vollständigen oder teilweisen Verseifung und/oder einer Hydrogenolyse unterwirft und in dieser Weise in die Verbindung der Formel (I) umwandelt.

10. Pharmazeutische Zubereitung enthaltend als Wirkstoff mindestens eine Verbindung nach einem der Ansprüche 1 bis 8 in Kombination mit einem inerten, nichttoxischen, therapeutisch verträglichen Bindemittel oder Trägermaterial.

**Anspruch** (für den Vertragsstaat AT)

Verfahren zur Herstellung der Verbindungen der allgemeinen Formel :

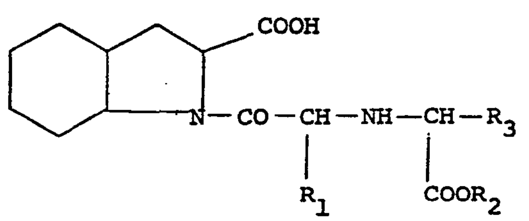

(I)

in der

$R_1$ eine Niedrigalkylgruppe mit 1 bis 4 Kohlenstoffatomen,

$R_2$ ein Wasserstoffatom oder eine Alkylgruppe mit 1 bis 4 Kohlenstoffatomen,

$R_3$ eine geradkettige oder verzweigte Alkylgruppe oder eine Mono- oder Dicycloalkylalkylgruppe, wobei diese Gruppen jeweils höchstens und insgesamt 9 Kohlenstoffatome aufweisen, oder eine substituierte Alkylgruppe der Formel :

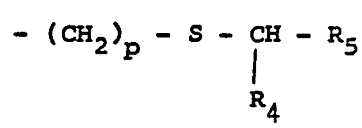

worin

$R_4$ ein Wasserstoffatom, eine Niedrigalkylgruppe ($C_1$-$C_4$) oder eine Cycloalkylgruppe ($C_3$-$C_6$),

$R_5$ ein Wasserstoffatom, eine Niedrigalkylgruppe ($C_1$-$C_4$), eine Cycloalkylgruppe ($C_3$-$C_6$) oder eine Alkoxycarbonylgruppe und

p 1 oder 2 darstellen, in Form des Racemats oder der optischen Isomeren und ihrer Salze mit einer anorganischen oder organischen, therapeutisch verträglichen Base oder ihrer Additionssalze mit einer

anorganischen oder organischen pharmazeutisch verträglichen Säure, dadurch gekennzeichnet, daß man einen Alkylester der Dicarbonsäure der allgemeinen Formel II :

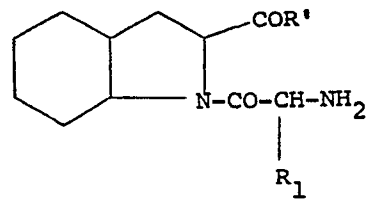

(II)

in der $R_1$ die bezüglich der Formel I angegebenen Bedeutungen besitzt und R′ eine Hydroxylgruppe oder eine Niedrigalkoxygruppe darstellt, einer reduktiven Alkylierungsreaktion mit einer Verbindung der allgemeinen Formel III :

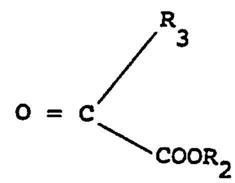

(III)

in der $R_2$ und $R_3$ die oben angegebenen Bedeutungen besitzen, zur Bildung eines Amins der allgemeinen Formel IV unterwirft :

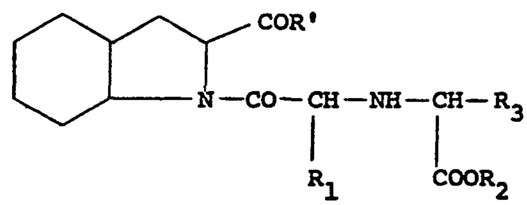

(IV)

in der R′ die oben bezüglich der Formel II angegebenen Bedeutungen besitzt und $R_1$, $R_2$ und $R_3$ die obigen Bedeutungen beibehalten, und nach der reduktiven Alkylierung die erhaltene Zwischenverbindung gegebenenfalls üblichen Verfahren zur Abspaltung der Schutzgruppe, wie beispielsweise einer vollständigen oder teilweisen Verseifung und/oder einer Hydrogenolyse unterwirft und in dieser Weise in die Verbindung der Formel (I) umwandelt.